(19)

Europäisches
Patentamt
European
Patent Office
Office européen
des brevets

(11) **EP 3 081 151 A1**

(12) **EUROPEAN PATENT APPLICATION**

(43) Date of publication:
**19.10.2016 Bulletin 2016/42**

(51) Int Cl.:
***A61B 5/00*** *(2006.01)*     ***A61B 5/16*** *(2006.01)*

(21) Application number: **15163865.7**

(22) Date of filing: **16.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Christensen, Finn Gilling**
**2920 Charlottenlund (DK)**

(72) Inventor: **Christensen, Finn Gilling**
**2920 Charlottenlund (DK)**

(74) Representative: **ZBM Patents ApS**
**Symbion Box:33**
**Fruebjergvej 3**
**2100 Copenhagen Ø (DK)**

Remarks:
Claim 16 is deemed to be abandoned due to non-payment of the claims fee (Rule 45(3) EPC).

(54) **DEVICE AND METHODS FOR DIAGNOSING ADHD AND/OR AUTISM**

(57) A device for use in for instance diagnosing ADHD and/or autism and methods for diagnosing neurobehavioral disorders.

**EP 3 081 151 A1**

## Description

## Field of the invention

The present invention relates to a device for use in for instance diagnosing ADHD and/or autism.

## Background of the invention

Neurobehavioral disorders are composed of a large group of behavioral impairments seen in association with brain diseases. Neurobehavioral difficulties involve two primary categories: cognitive decline, including memory problems and dementia; and neuropsychiatric disorders, including neurasthenia (a collection of symptoms including difficulty concentrating, headache, insomnia, and fatigue), depression, posttraumatic stress disorder (PTSD), and suicide. Other CNS problems can be associated with motor difficulties, characterized by problems such as weakness, tremors, involuntary movements, incoordination, and gait/walking abnormalities.

Neurobehavioral disorders also include attention deficit hyperactivity disorder (ADHD), autism, and a variety of learning disabilities. ADHD is a syndrome of inattention, hyperactivity, and impulsivity. Autism is a disorder characterized by impaired social interaction, verbal and non-verbal communication, and restricted and repetitive behavior.

Attention-deficit/hyperactivity disorder (ADHD) is the most common neurodevelopmental disorder of childhood. Basic information about the prevalence of ADHD varies in recent reports as wide as 2%-18%. Epidemiologic studies document increasing trends in prevalence of ADHD and ADHD medication during the past two decades. Thus, for example in USA, the average annual increase in the percentage of children with all diagnoses of ADHD was 3% from 1997 through 2006 (Vital and health statistics, 2008, Series 10, Nr. 237; Akinbami et al., 2011). In 2007, 4.8% of all children aged 4-17 years were taking medication for ADHD and the rates of medicated ADHD increased through the years. This disorder is an overwhelming public health problem with substantial social and economic impact, exerting a significant effect on quality of life.

Developing an effective case definition is a critical step for conducting effective treatment for children with Neurobehavioral disorders. However, questioning and observation - the prevalent methods now in diagnostics of mental pathological conditions - take much time, and the results are greatly subjective. The availability of standardized tools is limited, observations are unstructured, and the procedure can vary depending on theoretical orientation and experience of the psychologist. ADHD, like many other psychiatric and medical conditions, is a clinical diagnosis without objective laboratory tests. No laboratory tests reliably predict ADHD (Rowland, 2002).

According to the online Merck Manual Professional Edition (http://www.merckmanuals.com) ADHD has been classified as a developmental disorder, although some experts consider it a disruptive behavior disorder. According to the Diagnostic and Statistical Manual of Mental Disorders, Fourth Edition Text Revision (DSM-IV-TR), there are 3 sub-types of ADHD: Predominantly inattentive (ADHD-I), also called primer attention deficit (ADD), Predominantly hyperactive-impulsive (ADHD-H) and Combined (ADHD-C). The predominantly hyperactive-impulsive type occurs 2 to 9 times more frequently in boys; the predominantly inattentive type occurs with about equal frequency in both sexes.

ADHD has no known single, specific cause. Potential causes include genetic, biochemical, sensorimotor, physiologic, and behavioral factors. Some risk factors include birth weight of less than 1500 g, head trauma, and lead exposure, as well as prenatal exposure to alcohol, tobacco, and cocaine. Fewer than 5% of children with ADHD have other symptoms and signs of neurologic damage. Increasing evidence implicates abnormalities in dopaminergic and noradrenergic systems with decreased activity or stimulation in upper brain stem and frontal-midbrain tracts.

Onset of symptoms and signs often occurs before age 4 and invariably before age 7. The peak age for diagnosis is between ages 8 and 10. Diagnosis is clinical and is based on comprehensive medical, developmental, educational, and psychological evaluations. According to DSM-IV-TR diagnostic criteria include 9 symptoms and signs of inattention, 6 of hyperactivity, and 3 of impulsivity. Diagnosis using these criteria requires that symptoms and signs occur in at least 2 situations (e.g., home and school) and be present before age 7. Diagnosis of the predominantly inattentive type requires at least 6 of the 9 possible symptoms and signs of inattention. Diagnosis of the hyperactive-impulsive type requires at least 6 of the 9 possible symptoms and signs of hyperactivity and impulsivity. Diagnosis of the combined type requires at least 6 symptoms and signs each of inattention and hyperactivity-impulsivity.

Differentiating between ADHD and other conditions can be challenging. Many ADHD signs expressed during the preschool years could also indicate communication problems that can occur in other developmental disorders (e.g., autism) or in certain learning disorders, anxiety, depression, or behavioral disorders. However, during later childhood, ADHD signs become more qualitatively distinct; children with the hyperactive-impulsive or combined sub-types often exhibit continuous movement of the lower extremities, motor impersistence (e.g., purposeless movement, fidgeting of hands), impulsive talking, and a seeming lack of awareness of their environment. Children with the predominantly inattentive sub-type may have no physical signs.

In addition to clinical assessment of neurobehavioral disorders different devises and apparatus have been designed for systematic analysis of normal or impaired sensory-based reaction of persons using standardized and reproducible measurement techniques.

Recently, the US Food and Drug Administration (FDA) has allowed marketing of the first medical device based on brain function to help assess attention-deficit/hyperactivity disorder (ADHD) in children and adolescents 6 to 17 years old. The device, called Neuropsychiatric EEG-Based Assessment Aid (NEBA) System, is based on electroencephalogram (EEG) technology, which records different kinds of electrical impulses (waves) given off by neurons (nerve cells) in the brain and the number of times (frequency) the impulses are given off each second. The NEBA System is a 15 - to 20-minute non-invasive test that calculates the ratio of two standard brain wave frequencies, known as theta and beta waves. The theta/beta ratio has been shown to be higher in children and adolescents with ADHD than in children without it. When used as part of a complete medical and psychological examination, the device can help confirm an ADHD diagnosis or a clinician's decision that further diagnostic testing should focus on ADHD or other medical or behavioral conditions that produce symptoms similar to ADHD. (http://www.fda.gov/NewsEvents/Newsroom/PressAnnouncements/ucm360811.htm). Although such a method may have certain advantages, including the possibility to direct measuring brain functions, high effectiveness and preciseness, the ability to record all of the information automatically, using technical equipment for measuring brain functions in subjects with a mental handicap can entail great difficulties and be very stressful for the subject. For example, ADHD children may have problems sitting calm during EEG measuring where recording only lasted three minutes (Ahmadlou et al., 2011).

The European patent, EP2364443, discloses systems and methods for measuring the magnitude of attention and motor activity disturbances in a subject and has form basis for the Quotient® System that measures micro-motion and analyzes shifts in attention state. The Quotient ADHD Test is based on responses to images on a computer screen or to certain sounds and analyzing the pattern of mistakes that the subject makes and intends to provide quantitative data on the brain functions associated with hyperactivity, impulsivity and inattention. However, the system disclosed in EP2364443 cannot classify subjects having ADHD into the 3 sub-types ADHD-I, ADHD-H and ADHD-C.

ADHD is associated with a low educational outcome, and clinically, it is characterized by inattention, hyperactivity and impulsivity (Kuntsi 2006). These basic symptoms indicate the presence of cognitive deficits in ADHD patients related to problems in a range of timing functions (Noreika et al., 2013) and it was observed that overall response times of ADHD children are typically both slower and more variable than those of control children. Moreover, it was found that the reaction time variability (RTV), also known as intra-individual variability (IRTV), is a ubiquitous and robust phenomenon in ADHD (Epstein et al., 2011). Even though that children with ADHD can react to stimuli as quickly as their matched controls they demonstrate lapses in attention and more frequent responses with excessively long reaction time thereby increasing mean and variation values in (Leth-Steensen et al., 2000; Hervey et al., 2006).

The European patent, EP1656885, discloses a device for accomplishing a neuropsychological test. However, EP1656885 does not disclose an apparatus or methods for diagnosing disorders such as autism and ADHD.

Thus, there is a need for other diagnostic apparatus or devises, for diagnosing neurobehavioral disorders such as e.g. autism and ADHD.

## Summary of the invention

A problem to be solved by the present invention is to provide a device for diagnosing neurobehavioral disorders including autism and ADHD as well as methods for classifying subjects having ADHD into their 3 subtypes; Predominantly inattentive (ADHD-I), Predominantly hyperactive-impulsive (ADHD-H) and Combined (ADHD-C).

The solution is based on that the present inventors have observed that when exposing young human subjects having ADHD and/or autism to tactile stimuli to their finger-tips, they respond in different manners than normal children. For example, ADHD diagnosed children both exhibit a different pattern of individual reaction time variability (IRTV) and in omissions errors. Thus, by adding a feature to a testing device allowing for detecting the presence or absence of the finger-tip on the pressure bottom, both the pattern of IRTV and omissions errors can be recorded. Analysis of the recorded data revealed that combining measurements of variation in the reaction time means, the ratio value and the pattern of omission errors can be used as markers for diagnosing ADHD and autism as well as methods for classifying subjects having ADHD into their three subtypes ADHD-I, ADHD-H and ADHD-C.

Accordingly, a first aspect of the present invention relates to a device comprising a house unit having (a) a pressure means (2) that is movable from a resting position with no pressure of a finger-tip to an activating position upon the pressure of a finger-tip of a human subject and back to the resting position when the pressure of the finger-tip is removed, (b) a pin means (3) located in an opening in the pressure means (2), moving independently of the pressure means (2) and capable of moving from a lowered position wherein the opening is not protruded to a raised position protruding the opening of the pressure means (2) to apply a tactile stimulus to the finger-tip when the finger-tip is placed on the pressure means (2), and (c) a touch means (4) associated with the pressure means (2), wherein the touch means (4) can detect whether the finger-tip is placed on the pressure means (2) or not.

A second aspect of the invention relates to a method of diagnosing a human subject with ADHD using an apparatus as claimed in claim 1 of EP1656885 B1, comprising the steps of:

a) measuring and recording the number of activations of the pressure means (2) in a specific time period,
b) measuring and recording the number of tactile stimuli to the finger-tip of the human subject in a specific time period,
c) correlating the recorded data of steps a)-b) and determine that the subject has ADHD.

A third aspect of the invention relates to a method of diagnosing a human subject with ADHD using the device of the first aspect and/or herein relevant embodiments thereof, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has ADHD.

Similarly a human subject such as a child with autism can be diagnosed by a method of the invention concerning diagnosing a human subject with autism using the device of the first aspect and/or herein relevant embodiments thereof, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has autism.

A fourth aspect of the invention relates to a method of diagnosing a human subject with Predominantly inattentive ADHD using the device of the first aspect and/or herein relevant embodiments thereof, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has Predominantly inattentive ADHD.

A fifth aspect of the invention relates to a method of diagnosing a human subject with Predominantly hyperactive-impulsive ADHD using the device of the first aspect and/or herein relevant embodiments thereof, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has Predominantly hyperactive-impulsive ADHD.

A sixth aspect of the invention relates to a method of diagnosing a human subject with Predominantly combined inattentive and hyperactive-impulsive ADHD using the device of the first aspect and/or herein relevant embodiments thereof, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),

d) correlating the recorded data of steps a)-c) and determine that the subject has Predominantly combined inattentive and hyperactive-impulsive ADHD.

A seventh aspect of the invention relates to a method of treating a human subject with Predominantly inattentive ADHD, Predominantly hyperactive-impulsive ADHD, or combined Predominantly inattentive and hyperactive-impulsive ADHD, comprising testing a human subject with a device of any one of claims 1-10, diagnosing the subject with Predominantly inattentive ADHD of claim 13, Predominantly hyperactive-impulsive ADHD of claim 14, or combined Predominantly inattentive and hyperactive-impulsive ADHD of claim 15, and administering a suitable drug, such as a combination of amphetamine and dextroamphetamine (Adderall®) for treatment of Predominantly inattentive ADHD; methylphenidat (Ritalin®), dexmethylphenidate (Focalin®) or atomoxetine (Strattera®) for treatment of Predominantly hyperactive-impulsive ADHD and dextroamphetamine (Adderall®), methylphenidat (Ritalin®), dexmethylphenidate (Focalin®) or atomoxetine (Strattera®) treatment of combined Predominantly inattentive and hyperactive-impulsive ADHD.

The advantages of the methods of the present invention in relation to other prior art methods are; simplicity in application in that the measuring procedure is not stressful because during the testing the human subject sits at the table, with nothing restricting the subjects movements (e.g. belts, supports etc.), and there are no electrodes or other equipment placed on the skin of subjects. The only place of contact between the subject and device is the pins and buttons for the fingers, which provide the stimuli and measure the response. The device can be easily transported and prepared for testing in a few minutes. The children participating in the experiments perceive the test procedure as a kind of game, and enjoy "playing" with the buttons. Moreover, the device of the present invention is cheap to produce and the data sets recorded during the testing's are fast and simple to analyze thereby resulting in a fast and low-cost diagnostic method.

Definitions

Prior to a discussion of the detailed embodiments of the invention is provided a definition of specific terms related to the main aspects and embodiments of the invention. All definitions of herein relevant terms are in accordance of what would be understood by the skilled person in relation to the herein relevant technical context.

As used herein, the term "house unit" is without limitation intended to encompass any box, case or chassis suitable for accommodating the components of the device. The house unit can be constructed from any suitable material having a proper stiffness such as steel, aluminium, plastic, wood or glass.

As used herein, the term "pressure means" is without limitation intended to encompass any button, plate, knob or switch that can be used to rest a finger-tip on and which can move to different positions, typically up and down. The pressure means may be formed as a circular disk of metal or plastic and may have a central recess for a pin. The diameter of the pressure means is roughly the size of a fingertip.

As used herein, the term "pin means" refers without limitation to pins, nails, rods and the like, which has an oval or a sharp point on one end which is intended to exert a light force on the finger-tip of the subject, and the opposite end may have any shape that fits in the house unit of the device of the present invention. The pin is typically made from metal or plastic and is shaped such that it only needs to exert a light force on the finger-tip for the subject to sense the stimulus of the pin.

As used herein, the term "touch means" refers without limitation to a device, screen, display, button, plate or panel having a touch sensitive surface that allows for detecting the presence of a finger-tip on the surface. A touch sensitive surface encompasses any surface configured to detect or sense a touch by a finger-tip and interpret that touch as an input to a computing device. The underlying technology of the touch sensitive surface may be for example based on resistive, capacitive, surface acoustic wave, infrared, optical imaging, dispersive signal technology, acoustic pulse recognition etc.

As used herein, the term "tactile stimulus" refers without limitation to a stimulus relating to, mediated by, or affecting the sense of touch. Tactile stimulation includes the activating of nerve signals beneath the skin's surface (e.g. of a finger-tip) that inform the body of texture, temperature, vibrations and other touch-sensations.

As used herein, the term "attention deficit hyperactivity disorder (ADHD)" refers without limitation to inattention, over-activity, and/or impulsiveness. ADHD, which is also referred to in the literature as Attention Deficit Disorder/Hyperactivity Syndrome (ADD/HS), is a condition (or group of conditions) characterized by impulsiveness, distractibility, inappropriate behavior in social situations and hyperactivity. Symptoms of ADHD often diminish with age, but about 65% of individuals with ADHD continue to experience significant symptoms in adulthood. This disorder can impair social function, learning and/or development and is therefore now recognized as a serious problem. It is further recognized that many children with ADHD go on to develop other comorbid conditions or social problems in adulthood. In clinical terms ADHD is diagnosed if any one of the three main clinical features of inattention, over-activity and impulsiveness, persists in two or more situations, e.g., in both a home and school environment (American Psychiatric Association (Diagnostic and Statistical Manual of Mental Disorders, Fourth Edition, DSM-IV).

As used herein, the term "autism" refers without limitation to a neurodevelopmental disorder The disorder is characterized by atypical interaction (i.e. lack of attachment, inability to cuddle or to form reciprocal relationships, avoidance of eye gaze); insistence on sameness (i.e. resistance to change, performance of rituals, intense attachment to familiar objects, stereotyped and repetitive behavior); speech and language problems (ranging from total muteness to delayed onset of speech to markedly idiosyncratic use of language); uneven intellectual performance. Symptoms begin in early childhood. The cause in most children is unknown, although evidence supports a genetic component; in some patients, the disorders may be caused by a medical condition. Diagnosis is based on developmental history and observation. Treatment consists of behavioral management and sometimes drug therapy. All children with autism have similar problems with interaction, behavior, and communication; however, the severity of the problems varies widely.

As used herein, the term "reaction time" refers without limitation to the time elapsed between tactile stimulus is sent to the finger until the pressure means has been activated by the finger-tip by. "Reaction time mean" is the average time of one data set.

As used herein, the term "ratio value" refers without limitation to the ratio of the signal transfer time in the intermediate and/or efferent to the signal transfer time in the afferent part of the reflex arc and can be calculated by the formula RaV = (FT/2)/(RT-FT/2).

Further objects of the present invention will become apparent in view of the present description, figures and claims.

Drawing description

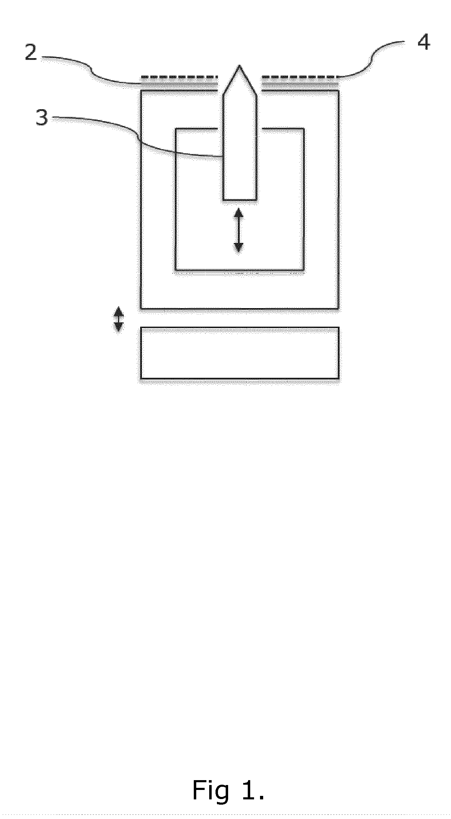

Figure 1A is a schematic illustration of a basic device of the invention.

Detailed description of the invention

As understood by the skilled person in the present context - it is understood to be most preferred to combine individual preferred embodiments described herein.

The present inventors have designed a device for testing or diagnosing ADHD and/or autism by measuring brain asymmetry through recording reactions to tactile stimuli onto finger-tips. These reactions provided information of signal transfer within one brain hemisphere and between hemispheres. In addition, reaction time variability (RTV), also called intra-individual variability (IRTV) can be determined. The device includes a pressure means (2), a pin located in an opening in the pressure means (2), that can apply a tactile stimulus to a finger-tip when the finger-tip is placed on the pressure means (2) and a touch means (4) associated with the pressure means (2), wherein the touch means (4) can detect whether the finger-tip is placed on the pressure means (2) or not. The characteristics of the individual components of the device, and their function in the context of the method of diagnosing a human subject with ADHD using the device will be explained in detail in the following. In a preferred embodiment the device is used for diagnosing ADHD or autism.

The device may contain one house unit or two house units, one for each hand, having one or several pressure means (2), pin means (3) and touch means (4) depending on whether the tactile stimulus has to be provided to one or more finger-tips on the hand. The a house unit of the device will typically be fabricated from suitable material having a proper stiffness such as steel, aluminium, plastic, would or glass. In a one embodiment the house unit is made from steel, aluminium or plastic. The house unit will typically only hold one pressure means (2), one pin means (3) and one touch mean (4) but may be constructed such that there are one pressure means (2), one pin means (3) and one touch means (4) for each of the five finger-tips. In another embodiment the house unit only contains one pressure means (2), one pin means (3) and one touch mean (4). In a preferred embodiment the device has 1 to 5 pressure means, such as 1.

The function of the pin means (3) is to provide tactile stimuli to finger-tips of a human subject. The subject is intended to respond to the stimuli by activating the pressure means (2), which records the action and sends a signal to a controller. The function of the touch means (4) that senses the presence of the finger-tip on the pressure means (2) is to record the presence of the finger-tip on the pressure means (2) and thereby determining if an omission in responding to a tactile stimuli to finger-tips is caused by lack of reaction to the stimuli or whether it is caused by absence of the finger-tip on the pressure means (2) such that the pin means (3) does not provide a tactile stimuli to the finger-tip. In other words, there are two different types of omission errors, one type is caused by lack of response to the tactile stimuli provided by the pin means (3), and another type is caused by the human subject has removed the finger-tip from the pressure means (2) when the pin means (3) is moved from the lowered position to the raised position protruding the opening of the pressure means (2) to apply a tactile stimulus to the finger-tip.

The pin means (3) is typically made from metal or plastic and is shaped such that it only needs to exert a light force on the finger-tip for the subject to sense the stimulus exerted by the pin. In one embodiment the pin means (3) is a stimulus pin. In another embodiment the pin is made from metal or plastic. In a further embodiment the tip of the pin means (3) has an oval shape.

Tactile stimulus is applied to a finger-tip by moving the pin located in the opening in the pressure means (2) from a lowered position wherein the opening is not protruded to a raised position protruding the opening of the pressure means (2). The force of the stimulus provided by the pin means (3) may be controlled by regulating the speed of the movement of the pin, the force by which the pin contacts the skin of the finger-tip, the duration of the stimuli or by the shape of the pin and by adjusting the height the pin protrudes over the surface.

Movement of the pin may be controlled by the use of an electromagnet, which is controlled, for example, via a relay, whereby the pin is forced up by the magnetic field upon activation of the electromagnet. Another way of controlling the movement of the pin may be by the use of hydraulic forces or by compressed air.

The pin means (3) may be connected to one of more sensors for detecting whether the pin is located in its lowered position or whether it is located in its raised position protruding the opening of the pressure means (2) to apply a tactile stimulus to the finger-tip. The pin means (3) may also be connected to one of more sensors allowing for measuring the position of the pin, the speed of the pin and time period taken for the pin to move its lowered position to its raised position protruding the opening of the pressure means (2). These sensors may either be constructed as individual sensors or as one common sensor capable for detection or measuring all described movements. In one embodiment the pin means (3) is connected to a first sensor for measuring when the pin means (3) is in the raised position. In a further embodiment the pin means (3) is connected to a second sensor for measuring the time period when the pin means (3) is in the raised position. In a still further embodiment the pin means (3) is connected to a further sensor for measuring the time period of moving the pin means (3) from its lowered position to its raised position protruding the opening of the pressure means (2). The skilled person knows which sensor types to select for the detecting and measuring the described operations of the pin means (3).

The standard parameters of the duration of the tactile stimulation may vary depending on the ADHD and/or autism to be diagnosed or on the human subject to be tested. In one embodiment the time period when the pin means (3) is in the raised position is from 1 to 10,000 msec, such as from 1 to 5,000 msec, such as from 1 to 2,000 msec, such as from 1 to 1,000 msec, such as from 1 to 500 msec, such as from 1 to 250 msec, such as from 1 to 100 msec, such as from 10 to 75 msec, such as from 15 to 50 msec, such as from 20 to 40 msec, such as from 25 to 30 msec. In a preferred embodiment the time period when the pin means (3) is in the raised position is 30 msec.

The standard parameters of the interval between successive stimuli may also vary depending on the ADHD and/or autism to be diagnosed or on the human subject to be tested. In one embodiment the time period between the pin means (3) is in the raised position is from 100 to 10,000 msec, such as from 500 to 8,000 msec, such as from 1,000 to 5,000 msec, such as from 2,000 to 3,000 msec such as from 200 to 300 msec such as from 20 to 30 msec. In a preferred embodiment the time period between the pin means (3) is in the raised position is from 2,000 to 3,000 msec.

The role of the pressure means (2) that is movable from a resting position with no pressure of a finger-tip to an activating position upon the pressure of a finger-tip, is to record and measure the response of the finger-tip to the tactile stimuli provided by the pin means (3).

The pressure means (2) may be in the form of a button, a plate, a knob or a switch that can be activated by pressure. The diameter of the pressure means is roughly the size of a fingertip. In one embodiment the pressure means is a pressure button or a pressure plate. In another embodiment, the pressure means may be formed as a circular disk of metal and/or plastic with a central recess for a pin.

The pressure means (2) may be constructed such that it includes or is connected to sensors that can detect the position of the pressure means (2) or the force exerted by the fingertip on the pressure means, in order to derive therefrom sensory-motor information. Accordingly, in one further embodiment, the pressure at which the pressure means (2) is operated by the finger-tip, can be measured, for example via the pressure means (2) being associated with a pressure sensor such as a piezo element.

Similarly, the pressure means (2) may also be connected to one or more sensors that allows for detecting whether the pressure means (2) is located in its resting position, the time the pressure means (2) is located in its resting position or in its activating position as well as the speed of movement of the pressure means and whether the finger-tip is placed on the pressure means (2) and the pressure applied to the pressure means (2) by the finger-tip of the subject. Thus, in one embodiment the pressure means (2) is connected to a third sensor for measuring when the pressure means is in the activating position. In a further embodiment the touch means (4) is connected to a fourth sensor for measuring when the finger-tip is placed on the pressure means (2). The skilled person knows which sensor types to select for the detecting and measuring the relevant operations of the pressure means (2).

The role of the touch means (4) is to detect the presence or absence of the finger-tip on the pressure means (2). The touch means (4) may be constructed as a device, screen, display or panel having a touch sensitive surface that allows for detecting the presence of a finger-tip on the surface. A touch sensitive surface encompasses any surface configured to detect or sense a touch by a finger-tip and interpret that touch as an input to a computing device. The underlying technology of the touch sensitive surface may be for example based on resistive, capacitive, surface acoustic wave, infrared, optical imaging, dispersive signal technology, acoustic pulse recognition etc. In one embodiment the touch means (4) is a screen, display or panel having a touch sensitive surface. The skilled person knows which touch sensitive screen, display or panel to select for the detecting and measuring the relevant operations of the touch means. In one embodiment of the present invention the touch means is connected to a fifth sensor for measuring the time period when the finger-tip is placed on the pressure means (2).

The operations performed by the pressure means (2) the pin means (3) and the touch means (4) may be recorded and sent to a controller. The controller is preferably designed such that, in addition to recording the operations of the pressure means (2), the pin means (3) and the touch means (4) the controller is also controlling the operation of the pin means (3) as a function of time, and preferably sends all the relevant data to a PC for further analysis using a suitable program developed for this purpose.

One way for constructing a device, similar to the device of the present invention but without the touch means (4) is disclosed in European patent EP1656885. Adding a touch means (4) as disclosed herein to the device of European patent EP1656885 will provide an embodiment of the device of the present invention.

The method for diagnosing ADHD and/or autism according to the present invention is based on the use of a device designed for measuring brain asymmetry through recording reactions to tactile stimuli onto fingertips. These reactions provide information of signal transfer within one brain hemisphere and between hemispheres. In addition, reaction time variability (RTV), also called intra-individual variability (IRTV) can be determined. Correlating such values with the number of errors of responding to the stimuli can be used for diagnosing ADHD and/or autism.

The measuring procedure is not stressful because during the experiment, the subject sits at the table with nothing restricting his or her movements (belts, supports, etc.), and there are also no electrodes or other equipment placed on his or her skin. The only places for contacts between the subject and the device are the buttons for fingers that realize the stimulation and measure the response.

The test method of diagnosing a human subject with ADHD and/or autism including autism and ADHD includes different testing procedures.

In one testing procedure, called the finger tapping (FT) procedure, the human subject repeatedly press a button on the device as quickly as possible, for about 10 seconds, using the index finger of one hand. The same procedure may be performed using the index finger of the other hand and the FT procedure may be repeated from 7 to 15 times such as 12 times. The device records the number of finger taps for each hand during each 10 seconds period. The average time for one finger tap, t, can be calculated for the left and right index fingers as $t = 10 \cdot R / N$, where R is the number the procedure is repeated and N is the total number of finger taps.

Another testing procedure, called the tactile reaction time (TRT) procedure allows the rate of tactile processing within one hemisphere and interhemispheric transfer to be measured. In this TRT testing procedure there is used a device having two or more pressure means (2). The human subject place the index fingers on the pressure means (2) and as soon as tactile stimulation is applied to one finger, the subject must respond by activating the pressure means (2) using a given finger.

The TRT procedure may consist of several sessions wherein, in a first session, the human subject normally is asked to respond to the stimulation using only the right finger, no matter which finger the stimulation is applied to. In a second session, the participant is normally asked to respond to all signals using only the left finger, no matter which finger the stimulation is applied to. Subsequent sessions normally repeat the first and second sessions. The TRT test may include form 1 to 10 sessions involving 60 stimulations each, with a break in-between. Each session may take about 2 to 3 minutes in duration. The length of the break depended on the participant.

At least the following information about each finger tap is recorded: (a) the finger the tactile signal was sent to, (b) the finger that was supposed to respond, (c) the finger that actually responded, (d) the reaction time (i.e. the time elapsed between the end of the tactile stimulus and the time the pressure means (2) was activated and the presence or absence of the finger-tip on the pressure means (2).

Diagnosing ADHD and/or autism according to the third, fourth, fifth and sixth aspect of the present invention is based on correlating data including reaction times, unilateral processing time, bilateral processing time and the number and pattern of errors made by the subject.

Unilateral processing is the rate of tactile processing within one hemisphere can be evaluated when the tactile stimulation and response are performed on the same side (i.e. when the stimulation is applied to one hand and the same hand performs the response). The response time for the left-sided condition (L/L) indicates the rate of right hemispheric processing whereas response time for the right-sided condition (R/R) indicates the rate of left hemispheric processing. The difference R/R - L/L is considered as an asymmetry index.

Bilateral processing of tactile stimulation can be evaluated when the stimulation is applied to one hand, but the other hand performs the response (R/L and L/R). This procedure is able to measure interhemispheric transfer time by calculating the difference in the response time between the unilateral condition and the bilateral condition. For example, the difference in the response time between the R/L and L/L conditions indicates the interhemispheric transfer time from the left to the right hemisphere. Correspondingly, the difference in the response time between the L/R and R/R conditions indicates the interhemispheric transfer time from the right to the left hemisphere.

A general behavior of subjects diagnosed with ADHD is that the frequency of errors is significantly higher than for control subjects. Errors can either be due to lack of response to a tactile stimulus, a wrong finger is responding the tactile stimulus (omission errors), or a finger is responding without a tactile stimulation onto the finger-tip (commission errors). The number of omission errors in normal children is less than 50% of all errors they make. This number is higher than 70% in children with ADHD.

Diagnosing Predominantly inattentive (ADHD-I), also called primer attention deficit (ADD) is based on evaluation of the average duration time of a response to a tactile stimuli obtained from the FT test divided by 2 (FT/2) for both hands. The TRT test sessions provide data of four different stimuli-reactions such as; stimuli on left finger-tip/response with left finger-tip (left/left or just L/L); stimuli on right finger-tip/response with right finger-tip (right/right or just R/R); stimuli on left finger-tip/response with right finger-tip (left/right or just L/R) and stimuli on left right-tip/response with right left-tip (right/left or just R/L). Then the response time for each of the different stimuli-reactions are calculated and the mean values obtained. The average duration time (FT/2) is subtracted from each of the stimuli-reaction means (FT) and the coefficients of variation of the stimuli-reaction means are evaluated. Coefficients of variations higher than 0.50 for L/R and R/L, higher than 0.52 for L/L, and higher than 0.48 for R/R are marks of ADHD-I in combination with the number and pattern of errors made by the subject.

Diagnosing Predominantly hyperactive-impulsive (ADHD-H) is based on calculation of the ratio value (RaV) that is obtained by dividing the average duration time (FT/2) with the average duration time (FT/2) is subtracted from each of the stimuli-reaction means (RT) according to the formula:

$$RaV = (FT/2)/(RT\text{-}FT/2)$$

Where, RT is the reaction time means that can be measured by 4 different ways:

R/R, L/L, R/L and L/R and (FT/2) for the right finger (tRH) and for the left finger (tLH).

$$RaV=(1/2tRH)/(R/R\ \text{-}1/2\ tRH)$$

$$RaV=(1/2tLH)/(L/L\ \text{-}1/2\ tLH)$$

$$RaV=(1/2tRH)/(L/R\ \text{-}1/2\ tRH)$$

$$RaV=(1/2tLH)/(R/L\ \text{-}1/2\ tLH)$$

RaV values lower than 0.22 for R/R, lower than 0.33 for L/L and lower than 0.25 for L/R and R/L is a mark of ADHD-H in combination with the number and pattern of errors made by the subject.

Diagnosing Predominantly combined inattentive and hyperactive-impulsive ADHD (ADHD-C) is based on coefficients of variations higher than 0.50 for L/R and R/L, higher than 0.52 for L/L, and higher than 0.48 for R/R are marks of ADHD-I and RaV values lower than 0.22 for R/R, lower than 0.33 for L/L and lower than 0.25 for L/R and R/L in combination with the number and pattern of errors made by the subject.

Diagnosing autism can be performed as described by Wittling (Wittling et al Behavior Research Methods 2009, 41 (3), 812-819). First, the rate of tactile processing within one hemisphere can be obtained from the sessions where the tactile stimulation and response are performed on the same side (L/L and R/R) and the corresponding Ft/2 is subtracted. Secondly, the inter hemisphere transfer time can be obtained from the sessions where the tactile stimulation and response are performed on opposite sides (L/R and R/L) and the corresponding Ft/2 is subtracted. Then, the difference in time between the unilateral condition and the bilateral condition is determined, i.e. the difference between the R/L and L/L conditions indicates the interhemispheric transfer time from the left to the right hemisphere and the difference in response time between the L/R and R/R conditions indicates the interhemispheric transfer time from the right to the left hemisphere. A difference of R/R - L/L higher than 50 msec and a difference (R/L - L/L) - (L/R - R/R) higher than 20 msec is a mark of autism.

**Detailed drawing description**

The following non-limiting drawing descriptions are for example purposes only.

A device of the invention in in its basic form is illustrated schematically in Figure 1, which shows a pressure means (2), a pin means (3) located in an opening in the pressure means and a touch means (4) associated with the pressure means (2). The pressure means (2) can move from a resting position to an activating position when pressure is applied to it by a subject and back to the resting position when the pressure is removed.

The pin means (3) located in an opening in the pressure means can move independently of the pressure means (2) and is capable of moving from a lowered position wherein the opening is not protruded the pressure means to a raised position protruding the opening of the pressure means (2) to apply a tactile stimulus to the finger-tip of a subject when the finger-tip is placed on the pressure means (2). The touch means (4) associated with the pressure means (2) can detect whether the finger-tip is placed on the pressure means (2) or not thereby allowing for detecting if the subject responds incorrectly.

**Examples**

Testing device

The device contains two identical panels, 23 x 18 x 4.7 cm in size, with 18 mm wide buttons for the fingers of each hand. The buttons have two functions. They can apply a slight tactile stimulation to a finger, and they can be depressed by the finger to provide a motor response to tactile stimulation, allowing for measurement of the response time. There is a small 5 mm wide hole in the middle of each button. A smooth metal pin that can provide a lightly touch to a finger through the hole performs the tactile stimulation. All parts of the device can be easily placed on a normal working table. The total weight of the device without the accompanying notebook computer is 732 gr. It can be easily transported and prepared for use in about 10 minutes.

The device is controlled by special software. It is possible to set the time interval between successive stimuli and their sequence. The test parameters are set up before the experiment and stored in the software. The test procedure is performed and recorded automatically, and the operator is unable to change the test parameters or the records during testing.

Test procedures.

At the beginning of the test, each child was given brief oral information about the device, and was then allowed to exercise using the buttons for a few minutes. The testing started with the FT test, followed by the TRT test. The whole procedure took about 30 minutes. One technician performed the ZenseTac tests on both ADHD and control children. Then all the individual data samples, without identification, were sent to another technician who analyzed them.

All tests were performed in a consulting room in the psychiatric clinic or in an isolated room at school.

*Finger tapping test (FT).*

A child is asked to repeatedly press a button on the panel as quickly as possible for 10 seconds using the index finger of one hand. After a break of 5 seconds, the same procedure is performed using the index finger of the other hand. This procedure is repeated 12 times. The device records the mean number of finger taps for each hand during each 10 seconds period. The average time for one finger tap, t, is calculated for the left and right index fingers (as a measure of manual motor speed) as: $t = 10 \cdot 12 / N$, where $N$ is the total number of finger taps.

*Tactile reaction time test (TRT).*

This test allows the rate of tactile processing within one hemisphere and interhemispheric transfer to be measured. The child is asked to focus attention on the index fingers and keep them on the buttons. As soon as tactile stimulation is applied to one finger, the child must respond by pressing the button using a given finger.

The tests consisted of four sessions involving 60 stimulations each, with a break between. Each session was three minutes in duration. The length of the break depended on the participant. The next session started when the child said he/she was ready to continue. Duration of the tactile stimulation was 30 msec and the interval between successive stimuli changed in random order from 2000 to 3000 msec. The tactile stimulation was performed using a single signal applied in a random order to the left (L) or right (R) finger in all the sessions. In the first session, the participant was asked to respond to the stimulation by pressing the button only with the right finger, no matter which finger the stimulation was applied to. In the second session, the participant was asked to respond to all signals using only the left finger. The third and fourth sessions were similar to the first two. Wrong reactions were excluded from the samples, but the number and type of errors were registered (wrong finger tap, missing finger tap, unnecessary finger tap). Since the right and left index fingers have different manual motor speeds, the time for one finger tap, t, using the given finger (determined during the FT test), was divided in two and subtracted from the response time. The time, t, was divided in two because the finger makes two rhythmic movements (up and down) during the FT test, within time t. In the TRT test, the reaction time includes only the down movement.

Evaluation of unilateral and bilateral processing of tactile stimulation.

*Unilateral processing.*

The rate of tactile processing within one hemisphere can be evaluated when the tactile stimulation and response are performed on the same side. The response time for the left-sided condition (L/L, signal sent to left finger and left finger responds) indicates the rate of right hemispheric processing whereas response time for the right-sided condition (R/R signal sent to right finger and right finger responds) indicates the rate of left hemispheric processing. The difference R/R - L/L was considered as asymmetry index.

*Bilateral processing.*

Bilateral processing of tactile stimulation can be evaluated when the stimulation is applied to one hand, but the other hand performs the response (R/L, signal sent to right finger and left finger responds) and (L/R, signal sent to left finger and right finger responds).

Statistical analysis.

The Intraclass Correlation Coefficient "Cronbach's alpha" was used to evaluate concordance between two diagnostic methods. Multivariative Analyses of Variance (MANOVA) and Post Hoc (Bonferroni) was used to assess difference between the groups. Comparisons between groups were also made using t-test and Mann-Whitney test. Pearson Correlation and Spearman's rho was used to evaluate correlations between the variables. Fisher's Test for Equality of Variances was used to evaluate difference in the variance for different variables. IRTV was assessed as coefficient of variation of reaction time.

Example 1

Participants.

Two groups of participants were tested. Children with ADHD, N=20 (16 boys and 4 girls) 7-11 years old medication naive children were recruited from Children's Psychiatric clinic in Denmark. The traditional method of ADHD diagnostics includes the patient history uptake, clinical observation, psychological study by WISC-III test and assessment of symptoms frequency and severity by questionnaire for parents. Children who yielded ICD-10 score F90.0 and had ADHD as the main diagnose were included in the research. As a note, autism was not included in the exclusion criteria in our study, and they were placed in the control group.

The control group (N= 18) consisted of children matching the ADHD diagnosed children in sex and age. They were recruited from an ordinary school and had normal skills. ADHD-rating scale, ADHD-RS, which is the only standardized scale for assessment of specific ADHD symptoms in Denmark (Du Paul GJ. et al. (1998) was used to ensure that none of the control children had ADHD symptoms.

Results

Both, ADHD and control children responded positively to the test procedure, and they were fully able to perform all the tasks.

The technician who analyzed the data samples from both the control group and the ADHD diagnosed children found IRTV values higher than critical values in 13 children, which could indicate that these 13 children have ADHD.

Since the number of children in the ADHD group was 20, the remaining 7 children with ADHD were not identified.

The data samples of the control group, was compared to data samples from the control group described in (Wittling et al, 2009) which were given the same test as the test used herein.

In this way it was found that 14 children out of 18 in the control group exhibited test parameters corresponding to the control group described in (Wittling et al, 2009), and these 14 children were recognized as real controls.

The remaining 11 children (7 from the ADHD group and 4 from the control group) had test results, which differed significantly from the standard, for example, too long reaction time, too high asymmetry indexes or too many errors in TRT test.

Since it is known that children with ADHD have, in average, a longer reaction time to rhythmical stimuli than children with typical development (Leth-Steensen, 2000; Epstein, 2011), the data samples exhibiting long reaction time were selected from the group of 12 children and analyzed. There were found 7 data samples having long reaction time, however, their manual motor speed measured in the FT test corresponded to the standard. This meant that they had long L/L and R/R values due to elongation of the afferent and/or central processing part of the reflex arc rather than long efferent part, i.e. slow movements of the fingers. The ratio of duration of the efferent part for afferent + central processing part of reflex arc was calculated:

$$(1/2tRH)/(RR-1/2tRH) \quad \text{and} \quad (1/2tLH)/(LL-1/2tLH),$$

where 1/2tRH and 1/2tLH is one second of time for one finger tap, *t*, determined in the FT test for the right and left hand correspondingly.

In what follows we denote these ratios RH/RR and LH/LL. For these 7 selected children, having long reaction but normal motor speed, time the ratio value (RaV) constituted 0,23±0,01 for RH/RR and 0,29±0,02 for LH/LL, which was significantly lower compared to 0,36±0,03 and 0,44±0,04 observed for the 14 children identified as controls (p< 0,01, t-test and Mann-Whitney test for both parameters).

There were 4 children left without identification. They had high index of brain asymmetry in the TRT test that was also observed in children with autism (Wittling, 2009), but both IRTV and RaV were within the normal range. As a note, autism was not included in the exclusion criteria in our study, and they were placed in the control group.

Conclusion

The results have shown that the above describe testing procedure identified 78% control children and 75 of the ADHD children correctly. However, our method did not find high IRTV or low RaV in five children in the ADHD group. The research protocol in our study did not permit a more thorough examination of children in case of a mismatch of the two diagnostic methods. We can just note that one of them contacted the psychiatric clinic because of fears provoked by some changes in his life. One other child had a note in the journal "not classic symptoms of ADHD" but they yielded ICD-10 score F90.0 and were recruited in the ADHD group.

Example 2

Example 1 above is repeated with the only difference that;

a) 40 children with ADHD, N=40 (30 boys and 10 girls) 7-11 years old medication naive children is recruited from Children's Psychiatric clinic in Denmark; b) the device of the present invention is used, and measurements of whether the finger-tip is placed on the pressure means or not, is recorded.

The date sets are analyzed by evaluating the following parameters for diagnosing ADHD and the 3 different sub-types:

*Predominantly inattentive (ADHD-I), also called primer attention deficit (ADD).*

The FT test protocol contains a number of measurements of the durations of each tap for the right and left fingers. We evaluate the average duration of one tap and divide it in two (FT/2) for both hands.
The TRT test protocol contains up to 240 reactions to four different stimuli reaction models: L/L, R/R, L/R and R/L. First, FT/2 from each of the four reaction time means is subtracted. Next, the coefficient of variation for the four reaction time means are evaluated. A coefficient of variation for the reaction time means higher than 0.50 for L/R, and higher than 0.52 for R/L, and higher than 0.48 for L/L is a mark of ADHD-I in combination with the number and pattern of errors made by the subject.

*Predominantly hyperactive-impulsive (ADHD-H)*

First the ratio value (RaV) of FT/2 and reaction time mean for the samples are evaluated.

$$RaV=(FT/2)/(RT-FT/2)$$

Where, RT is the reaction time means that can be measured by 4 different ways:

R/R, L/L, R/L and L/R and (FT/2) for the right finger (tRH) and for the left finger (tLH).

$$RaV=(1/2tRH)/(R/R -1/2\ tRH)$$

$$RaV=(1/2tLH)/(L/L -1/2\ tLH)$$

$$RaV=(1/2tRH)/(L/R -1/2\ tRH)$$

$$RaV=(1/2tLH)/(R/L -1/2\ tLH)$$

(RaV) lower than 0.22 for R/R, lower than 0.33 for L/L and lower than 0.25 for L/R and R/L is a mark of ADHD-H in combination with the number and pattern of errors made by the subject.

*Combined (ADHD-C)*

The children having a coefficient of variation for the reaction time means higher than 0.50 for L/R, and higher than 0.52 for R/L, and higher than 0.48 for L/L is and a RaV lower than 0.22 for R/R, lower than 0.33 for L/L and lower than 0.25 for L/R and R/L is a mark of ADHD-C in combination with the number and pattern of errors made by the subject.

**REFERENCES**

1: Vital and health statistics, 2008, Series 10, Nr. 237 p. 1-16.

2: Akinbami LJ, Liu X, Pastor PN, Reuben CA, (2011). NCHS Data Brif, 70, 1-7.

Rowland AS, Lesesne CA, Abramowitz AJ. (2002). The epidemiology of attention-deficit/hyperactivity disorder (ADHD): a public health view. Ment Retard Dev Disabil Res Rev;8(3):162-70..

Ahmadlou M, Adeli H. Fuzzy synchronization likelihood with application to attention-deficit/hyperactivity disorder. Clin EEG Neurosci. 2011 Jan;42(1):6-13.

Kuntsi J, McLoughlin G, Asherson P. 2006. Attention deficit hyperactivity disorder. Neuromolecular Med; 8(4):461-84.

Noreika V, Falter CM, Rubia K. 2013. Timing deficits in attention-deficit/hyperactivity disorder (ADHD): evidence from neurocognitive and neuroimaging studies. Neuropsychologia;51(2):235-66.

Epstein JN, Langberg JM, Rosen PJ, Graham A, Narad ME, Antonini TN, Brinkman WB, Froehlich T, Simon JO, Altaye M. 2011. Evidence for higher reaction time variability for children with ADHD on a range of cognitive tasks including reward and event rate manipulations. Neuropsychology, 25(4):427-41.

Leth-Steensen C, Elbaz ZK, Douglas VI.2000.Mean response times, variability, and skew in the responding of ADHD children: a response time distributional approach. Acta Psychol (Amst); 104(2):167-90. Hervey et al., 2006)

Wittling RA, Schweiger E, Rizhova L, Vershinina EA, Starup LB. 2009. A simple method for measuring brain asymmetry in children: application to autism. Behav Res Methods, 41(3):812-9.

**Claims**

1. A device comprising a house unit having (a) a pressure means (2) that is movable from a resting position with no pressure of a finger-tip to an activating position upon the pressure of a finger-tip of a human subject and back to the resting position when the pressure of the finger-tip is removed, (b) a pin means (3) located in an opening in the pressure means, moving independently of the pressure means (2) and capable of moving from a lowered position wherein the opening is not protruded to a raised position protruding the opening of the pressure means (2) to apply a tactile stimulus to the finger-tip when the finger-tip is placed on the pressure means (2), and (c) a touch means (4) associated with the pressure means (2), wherein the touch means (4) can detect whether the finger-tip is placed on the pressure means (2) or not.

2. The device of claim 1 for use in diagnosing ADHD or autism.

3. The device of claim 1 or 2, wherein the pressure means (2) is a pressure button or a pressure plate.

4. The device of any one of claims 1-3, wherein the device has 1 to 5 pressure means, such as 1.

5. The device of any one of claims 1-4, wherein the pin means (3) is a stimulus pin.

6. The device of any one of claims 1-5, wherein the pin means (3) is connected to a first sensor for measuring when the pin means (3) is in the raised position.

7. The device of any one of claims 1-6, wherein the pin means (3) is connected to a second sensor for measuring the time period when the pin means (3) is in the raised position.

8. The device of any one of claims 1-7, wherein the pressure means (2) is connected to a third sensor for measuring when the pressure means (2) is in the activating position.

9. The device of any one of claims 1-8, wherein the touch means (4) is connected to a fourth sensor for measuring when the finger-tip is placed on the pressure means (2).

10. The device of any one of claims 1-9, wherein the touch means (4) is connected to a fifth sensor for measuring the time period when the finger-tip is placed on the pressure means (2).

11. A method of diagnosing a human subject with ADHD using an apparatus as claimed in claim 1 of EP1656885 B1, comprising the steps of:

    a) measuring and recording the number of activations of the pressure means (2) in a specific time period,
    b) measuring and recording the number of tactile stimuli to the finger-tip of the human subject in a specific time period,
    c) correlating the recorded data of steps a)-b) and determine that the subject has ADHD.

12. A method of diagnosing a human subject with ADHD using the device of any one of claims 1-10, comprising the steps of:

    a) measuring and recording the number of activations of the pressure means (2) in a specific time period,
    b) measuring and recording the number of tactile stimuli to the finger-tip of the human subject in a specific time period,
    c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
    d) correlating the recorded data of steps a)-c) and determine that the subject has ADHD.

13. A method of diagnosing a human subject with Predominantly inattentive ADHD (ADHD-I) using the device of any one of claims 1-10, comprising the steps of:

    a) measuring and recording the number of activations of the pressure means (2) in a specific time period,
    b) measuring and recording the number of tactile stimuli to the finger-tip of the human subject in a specific time period,
    c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has Predominantly inattentive ADHD.

14. A method of diagnosing a human subject with Predominantly hyperactive-impulsive ADHD (ADHD-H) using the device of any one of claims 1-10, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has Predominantly hyperactive-impulsive ADHD.

15. A method of diagnosing a human subject with Predominantly combined inattentive and hyperactive-impulsive ADHD (ADHD-C) using the device of any one of claims 1-10, comprising the steps of:

a) measuring and recording the number of activations of the pressure means (2) in a specific time period,
b) measuring and recording the number of tactile stimuli to the finger-tip of the human subject in a specific time period,
c) measuring and recording the position of the finger-tip on the pressure means (2) and whether the finger-tip is resting on the pressure means (2) or not, and for how long time the finger-tip is resting on the pressure means (2), and for how long time the finger-tip is not in contact with the pressure means (2),
d) correlating the recorded data of steps a)-c) and determine that the subject has Predominantly combined inattentive and hyperactive-impulsive ADHD.

16. A method of treating a human subject with Predominantly inattentive ADHD, Predominantly hyperactive-impulsive ADHD, or combined Predominantly inattentive and hyperactive-impulsive ADHD, comprising testing a human subject with a device of any one of claims 1-10, diagnosing the subject with Predominantly inattentive ADHD of claim 13, Predominantly hyperactive-impulsive ADHD of claim 14, or combined Predominantly inattentive and hyperactive-impulsive ADHD of claim 15, and administering a suitable drug, such as a combination of amphetamine and dextroamphetamine (Adderall®) for treatment of Predominantly inattentive ADHD; methylphenidat (Ritalin®), dexmethylphenidate (Focalin®) or atomoxetine (Strattera®) for treatment of Predominantly hyperactive-impulsive ADHD and dextroamphetamine (Adderall®), methylphenidat (Ritalin®), dexmethylphenidate (Focalin®) or atomoxetine (Strattera®) treatment of combined Predominantly inattentive and hyperactive-impulsive ADHD.

2

4

3

Fig 1.

Europäisches Patentamt
European Patent Office
Office européen des brevets

## EUROPEAN SEARCH REPORT

Application Number

EP 15 16 3865

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/59377 A1 (BIOTHERAPEUTIC DEVICES INC [US]; UNIV NEW HAMPSHIRE [US]) 12 October 2000 (2000-10-12) * page 13, lines 11-23 * * page 16, line 18 - page 17, line 14; figure 4 * | 1-15 | INV. A61B5/00 A61B5/16 |
| X | JP 2010 022585 A (RION CO) 4 February 2010 (2010-02-04) * the whole document * | 1-15 | |
| X | EP 1 656 885 A1 (WITTLING WERNER DR [DE]; WITTLING RALF ARNE [DE]) 17 May 2006 (2006-05-17) * figure 3 * | 1-15 | |
| A | US 5 002 065 A (LACOURSE JOHN R [US] ET AL) 26 March 1991 (1991-03-26) * figure 2 * | 1-15 | |
| | | | TECHNICAL FIELDS SEARCHED (IPC) A61B |

The present search report has been drawn up for all claims

| Place of search | Date of completion of the search | Examiner |
|---|---|---|
| The Hague | 3 March 2016 | Lommel, André |

EPO FORM 1503 03.82 (P04C01)

## ANNEX TO THE EUROPEAN SEARCH REPORT
## ON EUROPEAN PATENT APPLICATION NO.

EP 15 16 3865

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2016

| Patent document cited in search report | | Publication date | Patent family member(s) | | Publication date |
|---|---|---|---|---|---|
| WO 0059377 | A1 | 12-10-2000 | AU | 4076200 A | 23-10-2000 |
| | | | WO | 0059377 A1 | 12-10-2000 |
| JP 2010022585 | A | 04-02-2010 | JP | 5270243 B2 | 21-08-2013 |
| | | | JP | 2010022585 A | 04-02-2010 |
| EP 1656885 | A1 | 17-05-2006 | AT | 366080 T | 15-07-2007 |
| | | | DE | 102004054656 B3 | 13-07-2006 |
| | | | EP | 1656885 A1 | 17-05-2006 |
| US 5002065 | A | 26-03-1991 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2364443 A
- EP 1656885 A
- EP 1656885 B1

**Non-patent literature cited in the description**

- **AKINBAMI et al.** *Vital and health statistics,* 2008
- Diagnostic and Statistical Manual of Mental Disorders
- Diagnostic and Statistical Manual of Mental Disorders. American Psychiatric Association
- **WITTLING et al.** *Behavior Research Methods,* 2009, vol. 41 (3), 812-819
- *Vital and health statistics,* 2008, (237), 1-16
- **AKINBAMI LJ ; LIU X ; PASTOR PN ; REUBEN CA.** *NCHS Data Brif,* 2011, vol. 70, 1-7
- **ROWLAND AS ; LESESNE CA ; ABRAMOWITZ AJ.** The epidemiology of attention-deficit/hyperactivity disorder (ADHD): a public health view. *Ment Retard Dev Disabil Res Rev,* 2002, vol. 8 (3), 162-70
- **AHMADLOU M ; ADELI H.** Fuzzy synchronization likelihood with application to attention-deficit/hyperactivity disorder. *Clin EEG Neurosci.,* January 2011, vol. 42 (1), 6-13
- **KUNTSI J ; MCLOUGHLIN G ; ASHERSON P.** Attention deficit hyperactivity disorder. *Neuromolecular Med,* 2006, vol. 8 (4), 461-84
- **NOREIKA V ; FALTER CM ; RUBIA K.** Timing deficits in attention-deficit/hyperactivity disorder (ADHD): evidence from neurocognitive and neuroimaging studies. *Neuropsychologia,* 2013, vol. 51 (2), 235-66
- **EPSTEIN JN ; LANGBERG JM ; ROSEN PJ ; GRAHAM A ; NARAD ME ; ANTONINI TN ; BRINKMAN WB ; FROEHLICH T ; SIMON JO ; ALTAYE M.** Evidence for higher reaction time variability for children with ADHD on a range of cognitive tasks including reward and event rate manipulations. *Neuropsychology,* 2011, vol. 25 (4), 427-41
- **LETH-STEENSEN C ; ELBAZ ZK ; DOUGLAS VI.** Mean response times, variability, and skew in the responding of ADHD children: a response time distributional approach. *Acta Psychol (Amst),* 2000, vol. 104 (2), 167-90
- **WITTLING RA ; SCHWEIGER E ; RIZHOVA L ; VERSHININA EA ; STARUP LB.** A simple method for measuring brain asymmetry in children: application to autism. *Behav Res Methods,* 2009, vol. 41 (3), 812-9